# EUROPEAN PATENT APPLICATION

(11) **EP 0 938 840 A1**
(43) Date of publication of application: **01.09.1999**
(21) Application number: 98941688.8
(22) Date of filing: 02.09.1998
(51) Int. Cl.: A01H 4/00, A01G 1/06

(54) **METHOD FOR PRODUCING VIRUS-FREE RED PEPPER PLANT SEEDLINGS**

(30) Priority: 08.09.1997 JP 24251597
(71) Applicant: Japan Tobacco Inc., Tokyo 105-8422 (JP)
(72) Inventor: KATO, Norio, Japan Tobacco Inc., Oyama-shi, Tochigi 323-0808 (JP); YUI, Mamiko, Japan Tobacco Inc., Oyama-shi, Tochigi 323-0808 (JP); YUASA, Hiroshi, Japan Tobacco Inc., Oyama-shi, Tochigi 323-0808 (JP)
(74) Representative: Reinhard - Skuhra - Weise & Partner
(86) International application number: JP9803938
(87) International publication number: WO9912412

(57) **Abstract**

A process for producing virus-free, young Capsicum annum plants wherein the cut surface of a section obtained by cutting a growing point off a Capsicum annum plant to be propagated is brought into contact with the cut surface of a raising stock obtained by cutting a separately and aseptically grown young Solanaceae plant, whereby said section is elongated on the cut surface of the raising stock, and from the elongated shoot, young Capsicum annum plants are raised. By this process, the growing point culture of Capsicum annum plants, particularly sweet pepper plants is made feasible for the first time. Further, according to this process, the production of virus-free, young Capsicum annum plants by vegetative propagation is made possible.

## Description

### TECHNICAL FIELD

The present invention relates to a process for producing virus-free, young Capsicum annum plants by vegetative propagation. In particular, the present invention relates to a process for producing virus-free, young Capsicum annum plants by vegetative propagation utilizing growing point culture techniques.

### BACKGROUND ART

By advance of cultural propagation techniques including growing point culture techniques as well as young plant propagation techniques such as grafting, cutting etc., young plants of even vegetables and flowering plants which have been produced from seeds can also be produced through vegetative propagation. Any of these techniques are important for vegetative propagation, and particularly growing point culture techniques are very important techniques for vegetative propagation as techniques for making plants virus-free and juvenile. These are essential techniques for crops whose final products have qualities and yields greatly influenced by viral infection.

The techniques for growing point culture of tomato have already been developed, and young plants can be produced by vegetative propagation, but for Capsicum annum L. plants which similar to tomato, belongs to Solanaceae, their growing-point culture and the in vitro propagation of their mature plants are difficult and have not been reported, and accordingly, production of virus-free young plants by vegetative propagation has also been impossible.

### PROBLEM TO BE SOLVED BY THE INVENTION

As described above, the growing point culture of Capsicum annum L. plants has been impossible heretofore. As a result of eager study for this problem, the present inventors enabled the growing point culture of Capsicum annum plants for the first time, to complete the present process for producing virus-free, young Capsicum annum plants.

The object of the present invention is to provide a process for producing young, virus-free Capsicum annum plants. In particular, the object of the present invention is to provide a process for producing the young of plants having spread as "sweet pepper" (also referred hereinafter to "sweet pepper plants") belonging to the Capsicum annum plant and bearing large sweet fruits. The other object of the present invention is to provide a process for producing young Capsicum annum plants on which juvenility has been conferred by growing point culture.

### DISCLOSURE OF THE INVENTION

The essence of the present invention lies in a process for producing virus-free, young Capsicum annum plants wherein the cut surface of a section obtained by cutting a growing point off a Capsicum annum plant to be propagated is brought into contact with the cut surface of a raising stock obtained by cutting a separately and aseptically grown young Solanaceae plant, whereby said section is elongated on the cut surface of the raising stock, and from the elongated shoot, young Capsicum annum plants are raised.

The process of the present invention comprises the step of obtaining a raising stock of Solanaceae plant, the step of obtaining a growing-point section of Capsicum annum plant, the step of obtaining a shoot having the growing-point section elongated therein, and the step of obtaining young Capsicum annum plants from the shoot.

First, the step of obtaining the raising stock of Solanaceae plant is described.

The Solanaceae plant as the raising stock is not particularly limited insofar as it is a plant belonging to the Solanaceae family. It is preferably a plant such as Capsicum baccatum, Capsicum chinense or Capsicum frutescens belonging to the same genus as Capsicum annum, more preferably the same plant as Capsicum annum L. to be propagated and most preferably a sweet pepper plant. The raising stock is obtained from an aseptically propagated Solanaceae plant. For raising the aseptically raised Solanaceae plant, disinfected seeds are sown in a medium used for plant tissue culture and sprouted in a culture chamber for tissue culture to raise the Solanaceae plant. Although the size of the raising stock obtained by raising the Solanaceae plant is not particularly limited, the Solanaceae plant having about one to three leaves developed two or more weeks after seeding is preferably used. The stem of the Solanaceae plant thus raised is cut with a sharp knife to give a raising stock. Although the position for cutting the stem is not particularly limited, the epicotyl over the cotyledon is preferably cut off. The cut surface of the raising stock may be horizontal but there are more preferable cases if the cut surface is inclined at about 45° relative to a horizontal plane. This is because if the cut surface is horizontal, water passing through the vessel may remain on the cut surface so that the growing-point section placed on the cut surface of the raising stock is washed off with said water.

Now, the step of obtaining the growing-point section of Capsicum annum plant is described.

Among Capsicum annum plants growing in a plastic house or a farm, the target Capsicum annum plant to be vegetatively propagated is selected and its growing point is removed. The method of removing the growing point is not particularly limited, and for example the following method is used. The tip of a branch is harvested from the growing Capsicum annum plant, and leaves are removed in a laboratory to facilitate removal of the growing point. For aseptic removal of the growing point, it may be disinfected by previously spraying ethanol. The growing point of the Capsicum annum plant, which is present in e.g. the base of an axillary bud, is cut and removed with a scalpel under a stereoscopic microscope. The size of the removed section containing the growing point is preferably 0.3 to 0.5 mm. This is not limitative. The target plant from which the growing point is removed has been confirmed to be virus-free, and if juvenility only is the object of growing point culture, the size of the section containing the growing point can also be further increased.

Now, the step of obtaining the shoot having the growing-point section elongated therein is described.

The growing-point section is placed on the cut surface of the above-described raising stock such that the cut surface of the growing-point section is brought into contact with the cut surface of the raising stock. The position for placing the growing-point section is preferably on a vascular bundle portion around the cut surface, and if the cut surface of the raising stock is inclined, the section is preferably placed on an upper part of the inclined surface.

If the growing-point section is thus placed on the raising stock and cultured for a predetermined period of time, preferably 1 to 3 weeks, the growing-point section is elongated to form a shoot. Meanwhile, if the growing point of the raising stock itself is elongated, it is removed preferably aseptically. The final step of obtaining the young Capsicum annum plant from the shoot is effected in a usual manner. For example, after the shoot obtained by elongating the growing-point section is cut into plural cuttings as necessary, each shoot cutting is dipped in a previously prepared medium for plant tissue culture and placed under suitable temperature, humidity and lighting conditions for growth, whereby its roots, stems and leaves can grow into a young Capsicum annum plant. Further, the young Capsicum annum plants thus obtained are subjected if necessary to acclimatization treatment so that they can be cultivated in a plastic house or a field.

Although the medium used in plant tissue culture, used in the process of the invention as described above, is not particularly limited, it is preferably a known MS medium, N6 medium or B5 medium, more preferably MS medium. Preferably, the medium is formed into a solid medium by adding agar, and as necessary, a wide variety of plant hormones can also be suitably added thereto.

These mediums used in plant tissue culture are used so that Capsicum annum plants serving as aseptic raising stocks are aseptically grown from Capsicum annum seeds, or shoots obtained by elongating the growing-point sections of Capsicum annum are dipped as cuttings in these mediums for aseptic growth whereby young Capsicum annum plants can be obtained.

### EXAMPLES

### [Example 1] Step of obtaining the raising stock of Solanaceae plant

Seeds from "Tosahikari D" (Nangoku Ikushu Kenkyu Nojyo Ltd.) and "KELVIN" (DeRuiter Seeds), which are varieties of sweet pepper plants belonging to the Capsicum annum L. plant, were surface-sterilized for 10 minutes with an aqueous solution of sodium hypochlorite at an effective chlorine concentration of 1 %. Then, the seeds were washed twice with sterilized water, and after excess water was absorbed into a filter paper, 1 seed was placed in each test tube containing a previously prepared medium containing various nutrient components sterilized in an autoclave. The medium was MS medium, in the preparation of which sucrose was added thereto at a concentration of 2 %, and after pH was adjusted to 5.8, agar was added thereto at a concentration of 0.8 %. After placed on the medium, each seed was grown in a culture chamber at 25 °C for a day length of 12 hours. About 3 weeks after culture was initiated, the plant arrived at the stage of growth utilizable as a raising stock. Just before the growing-point section as described in Example 2 was removed, the grown epicotyl was cut off with a sharp knife to give a raising stock. The surface to be cut was cut at an inclined angle of about 45° to the hypocotyl.

### [Example 2] Step of obtaining the growing-point section of Capsicum annum plant

To remove a growing-point section, a vigorously growing shoot was selected from a sweet pepper strain (improved strain F20005) growing in a plastic house and collected in a length of about 20 cm from the tip of a branch. For the purpose of facilitating the removal of the growing point after removal of leaves in a laboratory, its length was regulated within about 5 cm. In removing the growing point, no sterilization treatment was conducted, but if leave parasites were observed on the harvested shoot, the whole of the shoot was sprayed with about 70 % ethanol to prevent microbial contamination.

After the above treatment, a growing-point section of 0.3 to 0.5 mm in size was removed therefrom by use of a scalpel under a stereoscopic microscope in a clean bench. Growing points were found in the bases of many axillary buds, but those judged to be morphologically differentiated into flower buds were not removed.

### [Example 3] Step of obtaining the shoot having the growing-point section elongated therein

The growing-point section obtained in Example 2 was placed on the cut surface of the epicotyl of the raising stock prepared in the process of Example 1. In this step, the growing-point section was placed such that its cut surface was brought into contact with the cut surface of the raising stock. This operation was conducted under a stereoscopic microscope in a clean bench, and the position where the growing-point section was placed on the cut surface of the raising stock was around the cut surface of the epicotyl of the raising stock and on the vascular bundle in the upper part of the inclined surface. After the operation of placing the growing-point section on the cut surface of the raising stock, the test tube was immediately transferred to a culture chamber at 25 °C for a day length of 12 hours, and culture was continued. After the growing-point section was attached vitally to the cut surface of the raising stock, if the elongation of the growing point of the raising stock was initiated before the elongation of the growing-point section was initiated, the shoot elongated from the growing point of the raising stock was removed under aseptic conditions to promote the growth of the growing-point section.

Out of 30 growing-point sections placed on the raising stocks according to the above method, 2 growing-point sections were elongated to form shoots.

### [Example 4] Step of obtaining young Capsicum annum plants from the shoots

Once the shoot obtained by elongating the growing-point section placed on the cut surface of the raising stock was elongated to reach 2 to 3 cm, the shoot over the site in contact with the raising stock was cut off by means of a scalpel.

The cut shoot was dipped as a cutting in the medium for rooting. The media for rooting was prepared by adding IAA (indole acetic acid) at a concentration of 1 mg/l to the medium composition for aseptic seeding used in Example 1. The culture vessel used was an Agripot (Kirin Brewery Co., Ltd.) and 50 ml of the medium was introduced into each vessel. The transplanted shoot was observed to root after 1 week, and after 1 month, the shoot grew into a young sweet pepper plant of 5 cm in height. This sweet pepper was subjected to acclimatization treatment to become a healthy young sweet pepper plant capable of growing in a plastic house. The acclimatization treatment, in which the young sweet pepper plant obtained by culturing was covered with a plastic vessel to prevent evaporation of excess water, was conducted for 2 days under shielding conditions.

### [Example 5] Selection of varieties as the stock

By the means described in Examples 1 to 3, varieties other than the variety described in Example 1 were used as the stock, and the removed growing points were placed on the cut surfaces of hypocotyls from these varieties, to select a stock suitable for inducing elongation of shoots. As the stock varieties, California Wander (Sakatanotane K. K.), Wander Bell (Takii Shubyo K. K.), and Kyonami (Takii Shubyo K. K.) were used. Scion varieties, that is, the varieties whose growing point was removed and grafted onto the stock were California Wander, Kyonami and a selected strain (CU02SY) were used. Out of 50 growing points which were placed on the cut surface of hypocotyls of the stock plants, 16 shoots could be induced. Among these, California Wander could induce shoots at the highest degree, and the degree was 60 % (Table 1).

**Table 1**

| Relationship between Stock Varieties and Number of Induced Shoots | | | | |
|---|---|---|---|---|
| Stock variety | Scion variety | Number of grafts | Number of shoots | Efficiency (%) |
| California Wander | California Wander | 2 | 1 | 50.0 |
| | Kyonami | 13 | 10 | 76.2 |
| | Selected variety | 5 | 1 | 20.0 |
| | (subtotal) | 20 | 12 | 60.0 |
| Kyonami | California Wander | 3 | 0 | 0 |
| | Kyonami | 11 | 0 | 0 |
| | Selected variety | 5 | 0 | 0 |
| | (subtotal) | 19 | 0 | 0 |
| Wander Bell | Kyonami | 9 | 3 | 33.3 |
| | Selected variety | 2 | 1 | 50.0 |
| | (subtotal) | 11 | 4 | 36.4 |

### EFFECT OF THE INVENTION

According to the process of the invention as described above, the growing point culture of Capsicum annum plants, particularly sweet pepper plants was made feasible for the first time. According to the present invention, the production of virus-free, young Capsicum annum plants by vegetative propagation was made possible.

## Claims

1. A process for producing virus-free, young Capsicum annum plants wherein the cut surface of a section obtained by cutting a growing point off a Capsicum annum plant to be propagated is brought into contact with the cut surface of a raising stock obtained by cutting a separately and aseptically grown young Solanaceae plant, whereby said section is elongated on the cut surface of the raising stock, and from the elongated shoot, young Capsicum annum plants are raised.

2. A process for producing virus-free, young Capsicum annum plants according to claim 1 wherein the raising stock is a young Capsicum annum plant.

3. A process for producing virus-free, young Capsicum annum plants according to claim 1 or 2 wherein the elongated shoot is cut into plural pieces and then plural young Capsicum annum plants are raised therefrom.
